# FASCICULE DE BREVET EUROPEEN

(11) **EP 3 239 299 B1**
(45) Date de publication et mention de la délivrance du brevet: **07.11.2018**
(21) Numéro de dépôt: 17167680.2
(22) Date de dépôt: 24.04.2017
(51) Int. Cl.: C12P 3/00, C04B 41/50

(54) **PROCÉDÉ D'OBTENTION D'UNE SUBSTANCE MINÉRALE CIMENTAIRE**
VERFAHREN ZUM ERHALTEN EINER ZEMENTARTIGEN MINERALISCHEN SUBSTANZ
METHOD FOR OBTAINING A CEMENTITIOUS MINERAL SUBSTANCE

(30) Priorité: 25.04.2016 FR 1653614
(43) Date de publication de la demande: 01.11.2017
(73) Titulaire: Soletanche Freyssinet, 92500 Rueil-Malmaison (FR)
(72) Inventeur: SARRAF, Riad, 50460 Querqueville. (FR)
(74) Mandataire: Cabinet Plasseraud

(56) Documents cités:
- WO-A1-95/29250
- FR-A1- 2 430 727
- FR-A1- 2 723 080
- FR-A1- 2 948 224
- VARENYAM ACHAL ET AL: "Improved strength and durability of fly ash-amended concrete by microbial calcite precipitation", ECOLOGICAL ENGINEERING, ELSEVIER, AMSTERDAM, NL, vol. 37, no. 4, 7 novembre 2010 (2010-11-07), pages 554-559, XP028146959, ISSN: 0925-8574, DOI: 10.1016/J.ECOLENG.2010.11.009 [extrait le 2010-11-12]
- TINGTING ZHU ET AL: "Potential application of biomineralization by Synechococcus PCC8806 for concrete restoration", ECOLOGICAL ENGINEERING, vol. 82, 1 septembre 2015 (2015-09-01), pages 459-468, XP055329067, AMSTERDAM, NL ISSN: 0925-8574, DOI: 10.1016/j.ecoleng.2015.05.017
- LE METAYER-LEVREL G ET AL: "Applications of bacterial carbonatogenesis to the protection and regeneration of limestones in buildings and historic patrimony", SEDIMENTARY GEOLOGY, ELSEVIER, AMSTERDAM, NL, vol. 126, 1 janvier 1999 (1999-01-01), pages 25-34, XP002448927, ISSN: 0037-0738, DOI: 10.1016/S0037-0738(99)00029-9

## Description

### Domaine technique

La présente invention concerne le domaine des substances minérales cimentaires et leurs procédés d'obtention biologique. La présente invention concerne également le domaine des matériaux auto-régénérants comprenant une substance minérale cimentaire et leurs procédés de fabrication.

### Art antérieur

Des procédés d'obtention de substances minérales faisant appel à un procédé biologique de production de matière minérale et à des supports minéraux sont connus, tels que ceux décrits dans les documents FR2723080 et FR2644475. Ces matières minérales sont d'origine naturelle ou artificielle ou encore une partie de celles-ci est d'origine naturelle et une autre partie d'origine artificielle.

Le terme « minéral » est à prendre ici au sens large et signifie « incluant dans sa composition au moins un minéral, notamment carbonaté », c'est-à-dire une matière généralement solide définie par sa composition chimique et l'agencement ordonné de ses atomes. La composition chimique d'un minéral est constituée majoritairement, substantiellement, voire exclusivement, d'éléments autres que le carbone.

Les termes « d'origine artificielle » signifient « qui est synthétisé par l'action de l'homme » par opposition à « d'origine naturelle » qui signifie « qui est synthétisée dans la nature ».

Ces substances minérales ainsi créées peuvent être utilisées pour remplacer et/ou réparer ou complémenter des supports minéraux tels que la pierre, notamment la pierre calcaire, dolomitique ou gypseuse, tout en présentant une structure, une apparence et une texture proches ou similaires à celles de ces derniers.

Ces procédés connus, notamment ceux décrits dans les documents FR 2723080 et EP 0708836), comportent notamment les étapes de :
a. préparation d'une base comprenant une quantité prédéterminée d'une matière minérale synthétisée par au moins une structure vivante ou au moins une partie de structure vivante, la structure vivante appartenant au règne végétal, animal, ou microorganique ; et
b. traitement de la ladite base de façon à la transformer en une substance minérale inactivée de texture prédéfinie en fonction de l'usage extemporané auquel elle est destinée, par exemple pour son utilisation en tant que matière première pour le blocage des déchets par cimentation, autrement appelée charge d'apport.

Dans ces documents, le terme de « base » désigne soit la matière minérale seule ou la combinaison de la matière minérale avec un substrat de base permettant son développement. La matière minérale peut être par exemple l'oxalate de calcium, le carbonate de calcium, la silice, etc.

L'expression « structure vivante » désigne, dans ces documents, toute structure cellulaire ou d'origine cellulaire (végétale, animale ou micro-organique), vivante et/ou résultant de la vie et/ou des composés d'origine biologique, cristallisés ou non, tels des enzymes, hormones, protéines, ADN ; ces structures vivantes étant minéralisatrices, minéralisées ou minéralisables (ou pétrifiantes), c'est-à-dire aptes à synthétiser, dans la nature ou artificiellement, une matière minérale (brevets FR 2723080 et EP 0708836).

Ces procédés connus, bien que donnant satisfaction dans leur principe, nécessitent dans certains cas d'être améliorés.

### Présentation de l'invention

Ainsi, la présente invention propose un procédé d'obtention d'une substance minérale à partir d'une base comportant une matière minérale, le procédé comprenant l'obtention de la base comportant une quantité prédéterminée de la matière minérale synthétisée par une structure vivante ou une partie de celle-ci,
caractérisé en ce que l'obtention de la base comprend la fourniture de la structure vivante et la fourniture d'au moins un microorganisme lactique susceptible de symbiose avec la structure vivante pour la synthèse de la matière minérale de la substance minérale.

L'adjonction d'un microorganisme lactique permet l'accélération du processus de minéralisation des substances minérales ainsi créées (*i.e.* production de la matière minérale contenue dans les substances minérales), d'en améliorer la minéralité et de les diversifier.

L'obtention de la base peut comprendre en outre la fourniture d'une substance nutritive, préférablement de carbonate de glycérol et/ou de dérivés de carbonate de glycérol.

La structure vivante et le microorganisme lactique peuvent être fournis ensemble de manière à former un complexe biologique.

Le microorganisme lactique peut être d'abord fourni puis la structure vivante.

L'obtention de la base peut comprendre en outre la fourniture d'un substrat de base adapté au développement de la structure vivante et/ou du microorganisme lactique et la mise au contact du substrat de base avec la structure vivante et le microorganisme lactique.

Le procédé peut comprendre en outre le nettoyage des surfaces du substrat de base en contact avec le microorganisme lactique par celui-ci ; la synthèse de la matière minérale par la structure vivante, le nettoyage et la synthèse pouvant avoir lieu simultanément ou successivement dans cet ordre.

Le procédé peut comprendre en outre l'inactivation de la base pour obtenir une substance minérale inactivée. L'invention propose également une composition minéralisatrice comprenant :
a. une structure vivante ou au moins une partie de structure vivante ;
b. au moins un microorganisme lactique ; et
c. une substance nutritive.
Cette composition peut en outre être formulée pour une utilisation extemporanée.

L'invention propose également une substance minérale comprenant :
a. - une structure vivante ou au moins une partie de celle-ci ;
b. - au moins un microorganisme lactique ; et
c. - une matière minérale synthétisée par la structure vivante ou la partie de celle-ci.

Cette substance minérale est susceptible d'être obtenue notamment à partir du procédé décrit ci-dessus.

La substance minérale peut comprendre en outre un substrat de base adapté à la prolifération de la structure vivante et/ou du microorganisme lactique.

La matière minérale, et éventuellement le substrat de base, peut être présente sous une forme poreuse avec des pores débouchant à l'extérieur de la substance minérale dans lesquels la structure vivante et le microorganisme lactique sont présents.

La structure vivante et/ou le microorganisme lactique peu(ven)t être inactivé(s).

Enfin, de manière plus générale, la présente invention propose l'utilisation d'une combinaison d'une structure vivante et d'un microorganisme lactique en symbiose l'un avec l'autre, comme agent de minéralisation dans un matériau auto-régénérant, le microorganisme lactique étant apte à sécréter de l'acide lactique ; et celle de la composition minéralisatrice ou de la substance minérale décrite ci-dessus comme agent réparateur dans un matériau auto-régénérant.

### Dessins

D'autres objectifs, caractéristiques et avantages apparaitront à la lecture de la description qui suit en référence aux dessins donnés à titre illustratif et non limitatif parmi lesquels :
la figure 1 est un organigramme montrant les différentes étapes du procédé selon l'invention ;
la figure 2 est une photographie en noir et blanc montrant une matière minérale sous forme de néo-cristaux labyrinthiques de calcite pouvant être obtenue grâce à la présente invention ;
les figures 3 et 4 sont des photographies montrant des variétés de matière minérale sous forme de bio-carbonates cristallins pouvant être obtenue grâce à la présente invention ;
la figure 5 est une photographie en noir et blanc d'une production minérale silicocarbonatée obtenue grâce à la mise en oeuvre du procédé selon l'exemple 1;
la figure 6 est une photographie montrant une matière minérale silicocarbonatée pouvant être obtenue grâce à la présente invention ;
les figures 7 et 8 sont des photographies montrant des matières minérales sous forme de dépôt cristallin d'oxalate de calcium pouvant être obtenue grâce à la présente invention ; et
la figure 9 est une photographie en noir et blanc d'un comblement de fissures de mortier selon la mise en oeuvre du procédé selon l'exemple 2.

### Description détaillé

### Procédé d'obtention d'une substance minérale

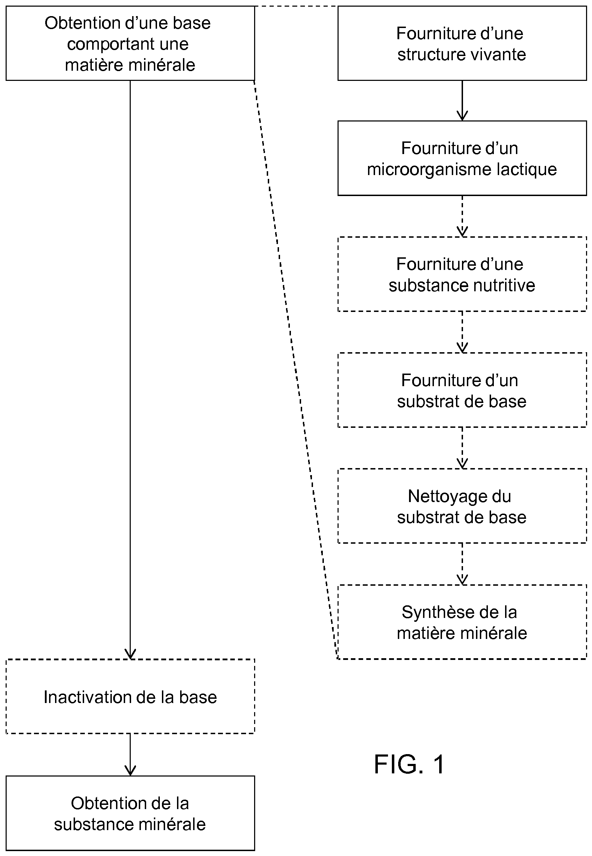

Un procédé d'obtention d'une substance minérale comprenant une matière minérale est décrit ci-après en référence à la figure 1.

Le terme « substance » désigne ici un matériau ou une composition.

Ce procédé comprend l'obtention d'une base comportant une quantité prédéterminée de la matière minérale synthétisée par une structure vivante ou une partie de celle-ci et la transformation de la base en la substance minérale. La matière minérale comprend notamment du calcaire ou du calcium. Par la suite, on se réfèrera uniquement à la structure vivante pour faciliter la lecture de ce présent exposé, mais les occurrences de ces termes doivent être comprises comme incluant également les modes de réalisation dans lesquels la structure vivante est remplacée par une partie de celle-ci, y compris pour les autres aspects de l'invention.

L'obtention de la base comporte la fourniture de la structure vivante et la fourniture d'au moins un microorganisme lactique susceptible de symbiose avec la structure vivante pour la synthèse de la substance minérale de la substance minérale.

Le terme « minéral carbonaté » est à prendre ici au sens large et signifie « incluant dans sa composition au moins un minéral », c'est-à-dire une matière généralement solide définie par sa composition chimique et l'agencement ordonné de ses atomes. La composition chimique d'un minéral est constituée majoritairement, substantiellement, voire exclusivement, d'éléments autres que le carbone autre que dans le carbonate.

Le terme de « base » désigne ici soit une matière minérale seule ou la combinaison d'une matière minérale avec un substrat de base permettant son développement. Lors de son développement sur le substrat de base, la matière minérale se substitue au moins en partie au substrat de base.

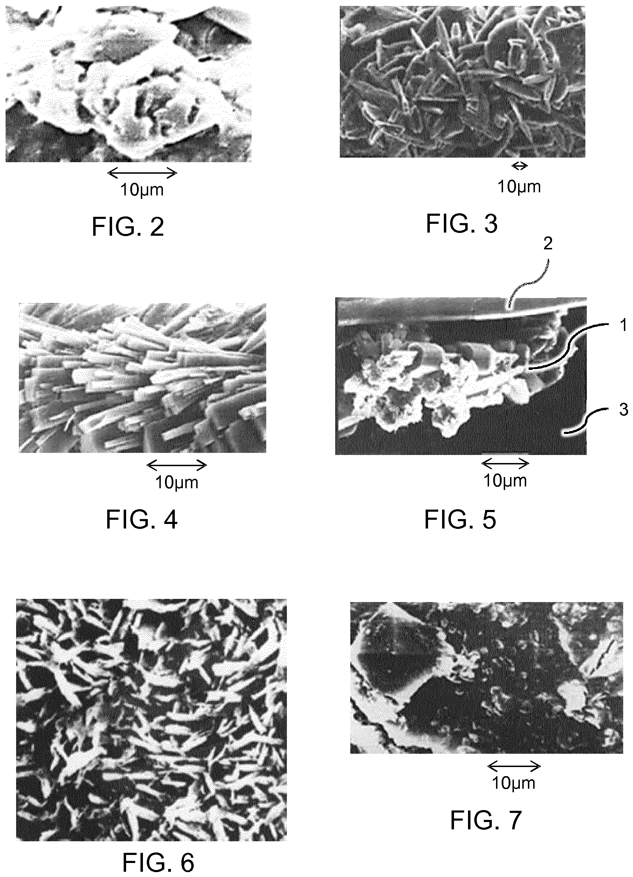

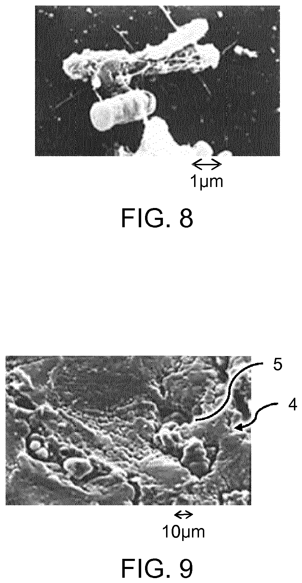

La matière minérale peut être par exemple de la calcite, des oxalates tels que l'oxalate de calcium, des carbonates (notamment des biocarbonates et des oligocarbonates) tels que le carbonate de calcium, de la silice, des silicocarbonates, etc. Les figures 2 à 8 montrent diverses matières minérales pouvant être obtenue.

Le terme de « symbiose » désigne, dans le cadre de la présente invention, une association intime, durable entre deux organismes (ici la structure vivante et le microorganisme lactique, les deux pouvant être qualifiés de symbiotes). Ainsi, les cas de relations parasitiques, *i.e.* dans lesquelles un des deux organismes tire un avantage tandis que l'autre subit les couts, ne sont pas compris.

Par conséquent, que ce soit pendant l'obtention de la base ou lors de l'utilisation de la substance minérale, le microorganisme lactique lors de la fermentation produit de l'acide lactique et du dioxyde de carbone servant de précurseur minéral pour la synthèse de la matière minérale par la structure vivante, notamment par minéralisation-carbonation organique qui est une minéralisation hybride. La matière minérale ainsi obtenue par minéralisation hybride s'en retrouve renforcée par rapport à une matière minérale obtenue sans adjonction de microorganisme lactique. En effet, la matière minérale, qui est à l'échelle moléculaire à la fois organique et minérale, est stable de par son insolubilité dans l'eau. Par ailleurs, la matière minérale présente une porosité ouverte.

L'expression « structure vivante » désigne, dans le cadre de la présente invention, toute structure cellulaire ou d'origine cellulaire (du règne des végétaux, celui des animaux, celui des Archées, celui des Bactéries, celui des Protistes ou celui des Mycètes), vivante et/ou résultant de la vie et/ou des composés d'origine biologique, cristallisés ou non, tels des enzymes, hormones, protéines, ADN ; ces structures vivantes étant minéralisatrices (capable de produire une matière minérale), minéralisées (dont au moins une partie est devenu une matière minérale) ou minéralisables (ou pétrifiante, *i.e.* dont au moins une partie est capable de devenir une matière minérale). Ainsi, elles sont aptes à synthétiser ou produire, dans la nature ou artificiellement, une matière minérale.

La structure vivante est préférablement une structure vivante calcifiée et/ou calcifiante (i.e. qui produit un minéral comprenant du calcium et/ou du magnésium).

La fourniture de la structure vivante peut être réalisée par culture, collecte (dans la nature).

Dans le cas où elle est cultivée, les conditions (par exemple durée et milieu de culture) de sa culture sont choisies pour permettre la synthèse de la matière minérale. De préférence, la structure vivante est cultivée jusqu'à atteindre sa phase exponentielle de croissance. La phase exponentielle de croissance peut être vérifiée, par exemple, par densité optique.

Dans le cas où elle résulte d'une collecte ou d'une récolte, la structure vivante est ensuite incorporée à la base.

Dans un mode de réalisation, la structure vivante est une ou plusieurs bactéries calcifiantes capables de minéralisation-carbonation selon par exemple le procédé décrit dans les documents FR2723080 et EP0708836. Des exemples de telles bactéries sont choisis parmi les genres *Bacillus* et *Pseudomonas.*

On entend par « microorganisme lactique », un microorganisme vivant (tel que des levures, des bactéries ou des virus) capable de s'associer par symbiose avec la structure vivante et/ou entre eux pour vivre et pour se développer, et capable de fermentation lactique, c'est-à-dire de produire de l'acide lactique sous condition anaérobie et en présence de glucides.

Dans le cas où il n'y a qu'un seul microorganisme lactique, celui-ci est capable de symbiose avec la structure vivante. En revanche, dans le cas où il y a plusieurs microorganismes lactiques, il n'est pas nécessaire que tous les microorganismes lactiques soient capables de symbiose avec la structure vivante, un microorganisme lactique avec une telle propriété suffit. Les microorganismes lactiques non capables de symbiose avec la structure vivante, le sont au moins entre eux.

Ces microorganismes lactiques agissent naturellement en symbiose et présentent des propriétés favorisant la synthèse de minéraux, notamment carbonatés. Ainsi ils améliorent le milieu dans lequel la structure vivante synthétise de la matière minérale en produisant du dioxyde de carbone grâce à la fermentation et de l'acide lactique. Les actions des microorganismes lactiques sont notamment :
a. la baisse du pH du milieu dans lequel la structure vivante synthétise la matière minérale ;
b. une digestion enzymatique des matières organiques, y compris les fibres ligneuses et cellulosiques, favorable à l'ancrage et la greffe de la matière minérale synthétisée par la structure vivante ;
c. le déplacement de l'équilibre calco-carbonique vers la production de dioxyde de carbone grâce à la fermentation induite par les microorganismes lactiques, ce qui est favorable à la synthèse de la matière minérale carbonatée par la structure vivante ; et
d. le nettoyage des surfaces d'un substrat de base préparant à l'ancrage de la matière minérale synthétisée par la structure vivante.

Afin de faciliter la lecture du présent exposé, il ne sera fait référence qu'à un seul microorganisme lactique. Cependant, cela doit être compris comme incluant les modes de réalisation dans lesquels plus d'un microorganisme lactique est utilisé dont au moins un est capable de symbiose avec la structure vivante, y compris pour les autres objets de l'invention.

Le microorganisme lactique peut être choisi parmi le groupe constitué de : la famille des Aérococcacées, la famille des Bifidobactériacées, la famille des Carnobactériacées, la famille des Enterococcacées, la famille des Lactobacillacées, la famille des Leuconostocacées, la famille des Saccharomycetacées, la famille des Sporolactobacillacées et la famille des Streptococcacées.

Préférentiellement, le microorganisme lactique est choisi parmi le groupe constitué de : la famille des Bifidobactériacées, la famille des Lactobacillacées, la famille des Saccharomycetacées et la famille des Streptococcacées.

Toujours préférentiellement, le microorganisme lactique est choisi parmi le groupe constitué de : la famille des Bifidobactériacées, la famille des Enterococcacées, la famille des Lactobacillacées, la famille des Leuconostocacées, la famille des Saccharomycetacées et la famille des Streptococcacées.

Parmi la famille des Bifidobactériacées, on peut citer *Bifidobacterium sp*., notamment *B. lactis, B. breve* et *B. bifidum*.

Parmi la famille des Enterococcacées, on peut citer *Tetragenococcus sp*., notamment *T. halophilus, T. muriaticus*, *T. solitarius*, *T. koreensis*, et *T. osmophilus.*

Parmi la famille des Lactobacillacées, on peut citer *Lactobacillus sp.* et *Pediococcus sp.*

Parmi la famille des Leuconostocacées, on peut citer *Leuconostoc sp.* et Oenococcus *sp*., notamment *L. mesenteroides* et *O*. *oeni.*

Parmi la famille des Saccharomycetacées, on peut citer *Saccharomyces sp*., notamment *S. cerevisiae* et *S. boulardii.*

Parmi la famille des Streptococcacées, on peut citer *Lactococcus sp.* et *Streptococcus sp*., notamment *L. lactis* (anciennement *S. lactis*).

L'obtention de la base peut en outre comprendre la fourniture d'une substance nutritive pour la structure vivante et/ou le microorganisme lactique. Cette substance nutritive peut notamment être choisie parmi le carbonate de glycérol, au moins un dérivé de celui-ci, ou un mélange de ceux-ci. Le dérivé de glycérol peut être par exemple un ester de glycérol.

La substance nutritive permet de nourrir la structure vivante et/ou le microorganisme lactique, ce dernier à son tour produit de l'acide lactique et du dioxyde de carbone lors de la fermentation fournissant à la structure vivante des sources de précurseurs de minéraux afin de perdurer la minéralisation-carbonation organique.

La substance nutritive peut être comprise dans un milieu de culture approprié et connu de l'homme du métier dans lequel la structure vivante et/ou le microorganisme lactique est cultivé. Le milieu de culture peut être un milieu liquide simplifié, notamment choisi parmi :
a. mélange aqueux comprenant 5 g de glucose et 10 g de fructose 10g, dans 1 L d'eau distillée ;
b. M.R.S. (gélose de Man, Rogosa, Sharpe), un milieu sélectif permettant des subcultures ; et
c. un mélange aqueux composé de 30 g de poudre gélose de Sabouraud dans 1 L d'eau distillée.

De préférence, le microorganisme lactique est cultivé jusqu'à atteindre sa phase exponentielle de croissance. La phase exponentielle de croissance peut être vérifiée, par exemple, par densité optique.

La structure vivante et le microorganisme lactique peuvent être fournis ensemble ou non, par exemple le microorganisme lactique est d'abord fourni puis la structure vivante.

La structure vivante et le microorganisme lactique forment ensemble un complexe biologique.

Le complexe biologique piège le dioxyde de carbone présent dans le milieu dans des bio-carbonates complexes qu'il produit et où il incorpore des protéines ayant une fonction de colle biologique. Ces bio-carbonates complexes sont similaires aux dépôts de minéraux naturels et présentent par leur teneur en silice une dureté accrue par rapport aux minéraux de silice et de calcium connus. Ces bio-carbonates complexes présentent par ailleurs un aspect et des caractéristiques cristallographiques et physicochimiques proches des matériaux minéraux naturels.

Dans le cas où la structure vivante et le microorganisme lactique peuvent être fournis ensemble, la culture de la structure vivante et celle du microorganisme lactique peuvent être réalisée dans un même milieu de culture, préférentiellement jusqu'à atteindre leur phase exponentielle de croissance.

Dans un tel cas, le milieu de culture peut comporter avantageusement de l'urée et du carbonate de glycérol pour favoriser le développement de types de minéralisation biologique extrêmement variés et la constitution d'une minéralisation biochimique par carbonatation organique créant des hybrides mêlant l'organique et le minéral à l'échelle moléculaire c'est-à-dire de nouveaux matériaux très stables grâce à des fonctions liantes spécifiques plus étendues.

Le concept de complexe biologique se comprend parfaitement bien dans le cas où le microorganisme lactique et la structure vivante sont fournis ensemble, mais ne ce réduit pas à cette situation. En effet, dans le cas où le microorganisme lactique et la structure vivante ne sont pas fournis ensemble, ils forment néanmoins également un complexe biologique dont les activités s'avéreront utiles mais de manière différée avant, pendant la synthèse de la matière minérale et lors de l'utilisation de la substance minérale.

L'obtention de la base peut encore comprendre la fourniture d'un substrat de base adapté au développement de la structure vivante et/ou du microorganisme lactique et la mise au contact du substrat de base avec la structure vivante et le microorganisme lactique.

Le terme « substrat de base » désigne toute matière ou matériau sur ou dans lequel la structure vivante et/ou du microorganisme lactique peuvent se développer.

Le substrat de base peut-être la pierre, notamment la pierre calcaire, dolomitique ou gypseuse. Le substrat de base peut être une partie d'un édifice existant ou un matériau de construction destiné à l'édification d'un bâtiment ou ouvrage.

Dans un tel cas, le procédé peut encore comprendre le nettoyage des surfaces du substrat de base en contact avec le microorganisme lactique par ce dernier et la synthèse de la matière minérale par la structure vivante, le nettoyage et la synthèse pouvant avoir lieu simultanément ou successivement dans cet ordre.

Le procédé peut en outre comprendre l'inactivation de la base pour obtenir une substance minérale inactivée. Le terme « inactivé » signifie ici « dénué de toute activité biologique », telle que d'une activité biominéralisatrice ou microbiologique pathogène.

L'inactivation de la base peut être obtenue par inactivation du microorganisme lactique et/ou de celle de la structure vivante, notamment après qu'une quantité suffisante de matière minéral a été synthétisée.

L'inactivation du microorganisme lactique et/ou de la structure vivante peut être réalisée par la mise en dormance de celui-ci par exemple par congélation, lyophilisation, cryodessiccation ou dessiccation. L'état de dormance désigne un état dans lequel le microorganisme lactique demeure vivant mais dans une léthargie caractérisée par un développement stoppé bien que le microorganisme reste en communication avec le milieu extérieur à la substance minérale inactivée. Le microorganisme lactique et/ou la structure vivante en état de dormance peut être revivifié(e/s). L'utilisation des formes revivifiables du microorganisme lactique et de la structure vivante permet d'obtenir des matériaux cimentaires autoréparateur et ouvre la voie aux superciments biologiques et aux pétrifiants à la prise, à la dureté et l'élasticité améliorées.

Alternativement ou en outre, l'inactivation de la structure vivante peut être réalisée par adjonction d'au moins un sel tel qu'un sel choisi parmi le groupe constitué de oxyde de magnésium (MgO), sulfate de magnésium (MgSO₄), phosphate (H₃PO₄), chlorure de calcium (CaCl₂), chlorure de baryum (BaCl₂), fluorosilicate de sodium (Na₂SiF₆), sel de carbonate (CO₃²⁻), silicate de sodium (Na₂SiO₃) et mélanges de ceux-ci. Le sel est avantageusement sous forme anhydre.

Alternativement ou en outre, l'inactivation de la structure vivante peut comprendre l'irradiation de celle-ci due à une activité radiologique externe et/ou l'élévation de température jusqu'à une température supérieure à celle que la structure vivante peut supporter. Dans le dernier cas, la structure vivante est morte et ne peut être réactivée.

Alternativement ou en outre, le procédé peut en outre comprendre une transformation de la base afin de conférer à la substance minérale inactivée une texture correspondant à l'usage auquel elle est prédestinée. Par exemple la transformation est le malaxage et/ou la fragmentation de la base afin d'obtenir une biomasse homogène qui est un mélange de la matière minérale avec la structure vivante et le microorganisme. Le malaxage et/ou la fragmentation sont réalisées au moins en partie simultanément à l'une des étapes décrites ci-dessus, et notamment l'inactivation de la base. La fragmentation peut être réalisée par exemple par broyage. Le malaxage peut être réalisé à l'aide d'un mélangeur connu de l'homme du métier. La fragmentation et/ou le malaxage permet d'obtenir la matière minérale sous forme d'une poudre et/ou d'amas granulaire.

La transformation peut en outre comprendre la dilution de la base, notamment dans un liquide, de préférence aqueux, tel qu'une solution nutritive pour la structure vivante et/ou le microorganisme lactique, notamment afin d'obtenir un blocage.

Le procédé peut en outre comprendre l'incorporation d'un agent de cohésion à la base. L'agent de cohésion et/ou de texture peut être choisi par exemple dans le groupe constitué de calcium, magnésium, silicium, baryum sodium, fer, manganèse, collagène, muco-polysaccharide, composé poly-cellulosique, et les mélanges de ceux-ci. à l'un au moins parmi la biomasse, le milieu, le matériau, la base et la matière minérale.

L'inactivation de la base et/ou le malaxage et/ou la fragmentation peuvent être réalisés successivement ou simultanément. De préférence, l'inactivation s'accompagne préférablement d'une transformation de la base inactivée.

La substance minérale peut être par exemple un matériau de construction, un sol renforcé. Le matériau de construction peut être obtenu à partir du recyclage d'un matériau de construction de départ ou de la réparation de ce dernier.

Le présent procédé peut être utilisé pour de multiples applications, telles que la construction confinée ou en environnement extérieur, le confortement des sols, l'agriculture ; et permet d'envisager quantité d'applications encore peu voire inconnues à ce jour, telles que la valorisation de déchets en provenance de matériaux de construction, la rénovation des surfaces artificielles, l'auto-réparation des mortiers, le comblement des microfissures, et l'induration des sols.

### Composition minéralisatrice

L'invention concerne également une composition minéralisatrice avantageusement utilisée pour réaliser le procédé décrit ci-dessus. La composition comprend la structure vivante, le microorganisme lactique et la substance nutritive tels que décrits ci-dessus.

La composition minéralisatrice peut comprendre 25 à 98 % en poids de la structure vivante, les bornes supérieures et les bornes inférieures préférées de cet intervalle pouvant être : 30 %, 70 %, 80 % et 95 %. Ainsi la quantité de la structure vivante peut préférentiellement être comprise dans n'importe quel intervalle dont les bornes supérieure et inférieure sont mentionnées ci-avant, notamment : 25 à 70 %, 30 à 70 %, 80 à 98 % et 95 à 98 % ; ou peut être égale à n'importe quelle valeur divulguée, éventuellement à ± 5 points de pourcentage.

La composition minéralisatrice peut comprendre 2 à 70 % en poids du microorganisme lactique, les bornes supérieures et les bornes inférieures préférées de cet intervalle pouvant être : 20 % et 30 %. Ainsi la quantité du microorganisme lactique peut préférentiellement être comprise dans n'importe quel intervalle dont les bornes supérieure et inférieure sont mentionnées ci-avant, notamment : 2 à 20 % et 30 à 70 % ; ou peut être égale à n'importe quelle valeur divulguée, éventuellement à ± 5 points de pourcentage.

La composition minéralisatrice peut comprendre 25 à 70 % en poids de la substance nutritive, les bornes supérieures et les bornes inférieures préférées de cet intervalle pouvant être : 30 % et 50 %. Ainsi la quantité de la substance nutritive peut préférentiellement être comprise dans n'importe quel intervalle dont les bornes supérieure et inférieure sont mentionnées ci-avant, notamment : 25 à 50 % et 30 à 70 % ; ou peut être égale à n'importe quelle valeur divulguée, éventuellement à ± 5 points de pourcentage.

Toutes combinaisons possibles des différents intervalles divulgués ci-dessus sont envisagées et notamment :
- 80 à 98 % ou 30 à 70 % en poids de la structure vivante et 2 à 20 % en poids du microorganisme lactique ;
- 30 à 70 % en poids du microorganisme lactique et 25 à 50 % en poids de la substance nutritive ; et
- 2 à 20 % en poids du microorganisme lactique et 30 à 70 % en poids de la substance nutritive.

La composition minérale est avantageusement formulée de manière à permettre une utilisation extemporanée. Elle peut subir une mouture et/ou une dilution pour favoriser son usage et être prête à l'emploi.

### Substance minérale

Une substance minérale selon l'invention, et susceptible d'être obtenue par le procédé précédemment exposé, est décrite ci-après.

Une telle substance minérale comprend :
a. une structure vivante ;
b. une matière minérale synthétisée par la structure vivante ; et
c. au moins un microorganisme lactique.

La quantité de structure vivante peut être comprise dans les mêmes intervalles que pour la composition minéralisatrice décrits ci-dessus.

La quantité de microorganisme lactique peut être comprise dans les mêmes intervalles que pour la composition minéralisatrice décrits ci-dessus.

Préférentiellement, la substance minérale comprend 25 à 70 % en poids de la matière minérale, les bornes supérieures et les bornes inférieures préférées de cet intervalle pouvant être : 30 %, 40 %, 50 % et 60 %. Ainsi la quantité de la matière minérale peut préférentiellement être comprise dans n'importe quel intervalle dont les bornes supérieure et inférieure sont mentionnées ci-avant, notamment : 30 à 70 %, 30 à 60 %, 40 à 60 % et environ 50 % ; ou peut être égale à n'importe quelle valeur divulguée, éventuellement à ± 5 points de pourcentage.

La quantité de substance nutritive peut être comprise dans les mêmes intervalles que pour la composition minéralisatrice décrits ci-dessus.

Toutes les combinaisons mentionnées ci-dessus pour la composition minérale sont aussi valables pour la substance minérale.

La substance minérale peut encore comprendre un support de base adapté à la prolifération de la structure vivante et ou du microorganisme lactique.

La substance minérale peut être active ou inactive.

La substance minérale peut se présenter sous forme solide telle qu'une forme pâteuse, gélifiée, pulvérulente, granuleuse (amorphe, cristallisée ou un mélange des deux). La substance minérale peut se présenter sous forme fluide, telle que sous forme liquide ou gazeuse, apte à se solidifier ou se condenser en un solide notamment par cristallisation dans un milieu poreux et/ou meuble tels les sols.

La substance minérale peut comprendre également au moins un sel tel qu'un sel choisi parmi le groupe constitué de oxyde de magnésium (MgO), sulfate de magnésium (MgSO₄), phosphate (H₃PO₄), chlorure de calcium (CaCl₂), chlorure de baryum (BaCl₂), fluorosilicate de sodium (Na₂SiF₆), sel de carbonate (CO₃²⁻), silicate de sodium (Na₂SiO₃) et mélanges de ceux-ci. Le sel est avantageusement sous forme anhydre.

Avantageusement, la substance minérale comprend 25 à 50 % en poids du sel, les bornes supérieures et les bornes inférieures préférées de cet intervalle pouvant être : 30 %, 40 % et 45 %. Ainsi la quantité de sel peut préférentiellement être comprise dans n'importe quel intervalle dont les bornes supérieure et inférieure sont mentionnées ci-avant, notamment : 25 à 50 % et 40 à 50 % ; ou peut être égale à n'importe quelle valeur divulguée, éventuellement à ± 5 points de pourcentage.

La substance minérale peut comprendre encore un agent de cohésion et/ou de texture.

Avantageusement, la substance minérale comprend 25 à 50 % en poids d'agent de cohésion et/ou de texture, les bornes supérieures et les bornes inférieures préférées de cet intervalle pouvant être : 30 %, 40 % et 45 %. Ainsi la quantité d'agent de cohésion et/ou de structure peut préférentiellement être comprise dans n'importe quel intervalle dont les bornes supérieure et inférieure sont mentionnées ci-avant, notamment : 30 à 50 % ; ou peut être égale à n'importe quelle valeur divulguée, éventuellement à ± 5 points de pourcentage.

La composition exacte de la substance minérale est choisie en fonction du substrat minérale sur lequel elle doit être appliquée. On pourra ainsi reconstituer le matériau des surfaces artificielles d'édifices endommagées par le temps selon leur nature cristallographique. La recréation de la « patine » de ce matériau s'en trouvera alors facilitée.

La structure vivante, le microorganisme lactique, la substance nutritive et l'agent de cohésion et/ou de texture sont tels que décrits ci-dessus.

La substance minérale est avantageusement formulée de manière à permettre une utilisation extemporanée.

La substance minérale peut être obtenue à partir d'une composition comprenant au moins la structure vivante et le microorganisme lactique. Des substances nutritives pour la structure vivante et/ou le microorganisme lactique sont préférentiellement comprises dans la composition. La composition peut être sous forme liquide plus ou moins visqueuse ou solide (par exemple sous la forme de granulés ou de poudre).

La matière minérale, et éventuellement le substrat de base, de la substance minérale est préférentiellement présente sous une forme poreuse avec des pores débouchant à l'extérieur de la substance minérale. En d'autres termes, le volume poreux est en contact fluidique avec l'environnement extérieur. Ce volume poreux constitue un espace dans lequel la structure vivante et le microorganisme lactique sont présents, notamment en état inactivés.

### Utilisation

La substance minérale décrite ci-dessus peut être utilisée comme charge, apport ou liant hydraulique dans un mélange pour comblement, ragréage, cimentation, assemblage, apprêt, revêtement, etc. Par exemple, un tel mélange comprenant la substance minérale peut être utilisé pour combler une cavité formée par l'érosion dans une roche calcaire.

Ainsi, la substance minérale peut encore être utilisée pour le blocage de déchets et/ou de sédiments de toute sorte, la restructuration des constructions, la régénération des mortiers et des bétons par comblement des fissures, le confortement des sols meubles poreux et/ou mouvants, la création de minéralisations à titre curatif, préventif ou décoratif.

On entend par le blocage de déchets, la possibilité de les contenir ou de les séquestrer dans un mélange réalisée à partir de la substance minérale obtenue par ce procédé.

De manière plus générale, la présente invention concerne l'utilisation d'une combinaison d'une structure vivante et d'un microorganisme lactique en symbiose l'un avec l'autre, comme agent de minéralisation, notamment dans un matériau de construction, par exemple un matériau auto-régénérant.

### Exemples

### Exemple 1

Cet exemple concerne le recyclage de matériau de construction, le matériau de construction formant un substrat de base. Le matériau de construction, par exemple du mortier portland, ou la partie de celui-ci, est préalablement concassé en fragments avantageusement centimétriques.

Dans ce procédé, la structure vivante, le microorganisme lactique et la substance nutritive sont fournis ensemble sous forme d'un bain aqueux riche en microorganisme lactique.

Le substrat de base est ensuite mis en contact avec ce bain aqueux permettant la lixiviation ou lessivage des surfaces du substrat de base grâce à l'action de l'acide lactique produit par le microorganisme lactique qui désagrège progressivement le substrat de base en surface en libérant des substances silico-carbonatées.

En d'autres termes, les couches superficielles du matériau de construction ou de la partie de celui-ci se dissolvent par un effet de mordançage pour aboutir à la destruction de leur structure cristalline. En outre, la lixiviation a un effet nettoyant des surfaces du substrat de base favorable à la minéralisation-carbonatation ultérieure ce qui permet de se passer d'une étape additionnelle de nettoyage, notamment par des procédés chimiques destructeurs et dangereux.

Le microorganisme lactique a été préalablement cultivé jusqu'à atteindre leur phase exponentielle de croissance. La durée de la culture nécessaire pour atteindre ce stade est généralement comprise entre 48 et 72 heures à une température de 20 °C pour un volume de 5 L de gélose de Sabouraud.

Conjointement à la lixiviation, le procédé peut comprendre le relargage des surfaces du substrat de base, assurant ainsi un lessivage progressif de ces surfaces. Le refoulage peut être réalisé entre quelques jours à plusieurs mois. Le refoulage est préférentiellement réalisé à l'aide d'une pompe aspirante refoulante permettant de mettre en mouvement le bain de lixiviation.

Par la suite, une structure vivante est fournie pour la minéralisation, ce qui permet d'aboutir à un matériau comprenant une matière minérale synthétisée par la structure vivante, éventuellement en mélange avec une autre matière minérale non synthétisée par la structure vivante.

Ainsi, il est possible de valoriser ces matériaux de construction autrement voués à la destruction ou destinés à la décharge. L'aspect de la matière minérale obtenue à partir d'un mortier de portland est montré sur la figure 2 sur la laquelle sont visibles :
a. la matière minérale **1**, ici silicocarbonatée ;
b. le substrat de base **2** ; et
c. la solution riche en structure vivante et microorganisme lactique **3**.

### Exemple 2

Cet exemple concerne le traitement de matériau de construction fissuré (comblement), notamment du mortier fissuré. Ce mortier fissuré forme alors le substrat de base.

Dans cet exemple, le microorganisme lactique (ici une bactérie lactique) et la substance nutritive sont fournis ensemble sous forme d'une solution aqueuse riche en microorganisme lactique en phase exponentielle de croissance.

Le substrat de base est mis au contact de la solution aqueuse permettant un nettoyage préalable de la paroi des fissures. La mise au contact est réalisée par application au pinceau ou au pulvérisateur de la solution aqueuse.

L'action nettoyante est rapide et après quelques heures (1 à 2 heures), la structure vivante est fournie et mise au contact avec le substrat de base par goutte à goutte d'un milieu de culture à l'urée comprenant, dans un volume d'un litre, 1 g de glucose, 10 g d'urée, 10 g de carbonate de glycérol, et la structure vivante minéralisatrice telle que *Bacillus megaterium* et/ou *Bacillus cereus* (bactéries calcifiantes), en phase exponentielle de croissance.

La structure vivante permet la reminéralisation progressive des fissures.

L'intérêt de la symbiose repose ici sur l'utilisation d'une bactérie lactique dont le rôle majeur est le nettoyage des parois des fissures à reminéraliser par sécrétion de l'acide lactique, et conjointement l'utilisation de bactéries calcifiantes pour le comblement des fissures.

À la fin du procédé, on constate le comblement des fissures par des productions biominérales variées notamment calciques et oxaliques visible sur la figure 3. Dans laquelle sont visibles :
a. le substrat de base fissuré **4** ; et
b. la matière minérale (calcique et oxalique) **5**.

Contrairement à un simple comblement qui laisse toujours une interface discontinue entre le matériau à réparer et le comblement, la réparation du matériau est ici plus résistante du fait que la lixiviation produite par le microorganisme lactique permet un meilleur ancrage de la matière minérale synthétisée par la structure vivante de sorte qu'il y a une continuité intime entre le matériau et la matière minérale.

Ainsi, sur des surfaces artificielles, telles que par exemple celles d'édifices en roches naturelles et/ou en mortiers et/ou en béton de toute spécificité, on réalise de cette façon des revêtements bio-cristallins divers : carbonates, oxalates, sulfates (rose des sables), phosphates (apatite), fluosilicate et on crée des matériaux nouveaux hybrides qui mêlent l'organique et le minéral à l'échelle moléculaire.

### Exemple 3

Cet exemple concerne le traitement de productions coralliaires, d'algues calcaires, de coquilles, appelées ci-après productions coquillères marines fraiches. Ces productions coquillères marines fraiches sont utilisées comme substrat de base. Le microorganisme, la structure vivante et la substance nutritive sont fournis ensemble sous la forme d'une solution aqueuse riche en microorganisme lactique en phase exponentielle de croissance.

Le substrat de base est soumis à un lessivage (lixiviation) par mise en contact avec la solution aqueuse. Le substrat de base subit alors une fermentation enzymatique qui se révèle antiseptique et qui agit au niveau des liaisons organiques/inorganiques de la composition des édifices biocristallins naturels compris dans le substrat de base.

Cette étape préparatoire procure au substrat de base une plus grande aptitude à la pétrification artificielle lors des étapes suivantes du procédé.

Ce procédé, qui comporte également la désodorisation de la matière minérale carbonatée obtenue, permet :
a. d'améliorer le recyclage des productions coquillères marines fraiches ; et
b. le nettoyage des surfaces artificielles dans le cadre de leur rénovation/reminéralisation.

Le procédé comporte en outre le traitement de surfaces artificielles à l'aide de la substance minérale obtenue. Ce traitement se révèle simple et peu couteux dans un domaine où les salissures nécessitent souvent l'emploi d'abrasifs destructeurs. Le procédé comporte enfin la reminéralisation des dépôts organiques et minéraux dus à la pollution effectuée par la structure vivante. Ainsi, il permet un gain de temps lors des interventions de rénovation/réparation. Les objets isolés tels les moellons, blocs de pierre de toute nature minéralogique ou matériaux de construction telle que brique, tuile, béton, ciment ou démontables tels les statuaires peuvent être soumis à ce procédé.

### Exemple 4

Cet exemple concerne le traitement de broyat et de broussailles. Des échantillons de broyat de broussailles sont utilisés comme substrat de base et sont plongés dans un bain aqueux riche en microorganisme lactique en phase exponentielle de croissance et comprenant une substance nutritive. Ces échantillons subissent une fermentation enzymatique digérant en partie les fibres cellulosiques, pecto-cellulosiques et ligneuses.

Ce procédé s'adresse tout particulièrement aux industries de la filière bois confrontées à la nécessité de destruction des fibres cellulosiques, pecto-cellulosiques et ligneuses en permettant rapidement leur digestion.

Par exemple, un échantillonnage de broyat de broussailles de quelques litres traités selon le procédé de la présente invention par immersion durant au minimum 72 heures et au plus 144 heures à la température de 20°C, a permis grâce à la prédigestion des fibres des broyats de broussailles, une fois séchés, une pétrification plus aisée sous l'action de la structure vivante par rapport par exemple aux procédés décrits dans FR 2723080 et EP 0708836.

### Exemple 5

Cet exemple concerne l'induration des sols. Des profils de sols de type alluvionnaire meubles reconstitués selon des cylindres de 4 centimètres de diamètre et de 10 centimètres de haut sont utilisés comme substrat de base et sont tout d'abord percolés par une solution aqueuse riche en microorganisme lactique en phase exponentielles de croissance et comprenant une substance nutritive.

Le procédé selon l'invention permet la préparation du sédiment à une pétrification maximale grâce à l'action primordiale de débusquement enzymatique opérée par le microorganisme lactique durant 72 heures.

Les profils de sols de type alluvionnaire meubles reconstitués sont secondairement infiltrés par une solution nutritive composée de glucose, urée et carbonate de glycérol contenant la structure vivante, notamment *Bacillus megaterium* et/ou *Baccilus cereus*, en phase de croissance exponentielle. Le milieu nutritif simplifié est constitué de 5 g de peptone, 10 g de glucose, 0,005 g de MgSO₄, 0,1 g de MgCO₃, 0,1 g de K₂CO₃, 0,1 g de Ca(NO₃)₂, 0,05 g de ZnSO₄ et de l'eau aux quantités suffisantes pour un litre.

On constate par ce procédé une induration progressive du sédiment par développement d'une sédimentation cristalline autour des grains alluvionnaires dès les premiers jours du traitement conduit, au total, pendant une semaine et aboutissant à une formation gréseuse solide au bout d'une quinzaine de jours d'assèchement naturel des éprouvettes.

La minéralisation classique par la structure vivante relevant de l'art antérieur est renforcée dans cet exemple par la minéralisation causée par la carbonatation organique résultant de l'activité du microorganisme lactique produisant du dioxyde de carbone, à partir de ce dioxyde de carbone, de l'urée et du carbonate de glycérol.

On constate par examen microscopique que l'induration progressive constatée expérimentalement résulte de la coalescence de de la matière minérale synthétisée par la structure vivante dans lesquels cette dernière et le microorganisme lactique sont en dormance mais revivifiables. Les biominéraux se présentent notamment sous des aspects morphoscopiques et endoscopiques spécifiques appelés labyrinthiques, c'est-à-dire qu'ils sont à la fois poreux et en communication fluidique avec le milieu extérieur permettant divers échanges (gazeux, liquides, vivants) et constituent un habitat pour la structure vivante et le microorganisme à l'état de dormance.

Les substances mucoprotéiques, silicocarbonatées constituants essentiels des collages biologiques de la matière minérale secrétée par la structure vivante sous forme cristalline participent à l'encapsulage de la structure vivante et du microorganisme lactique nettement favorisé par le carbonate de glycérol et/ou dérivés qui boostent l'intégration du carbone organique des mucoprotéines cachées, recombinantes, liées et des colles biologiques en général.

## Revendications

1. Procédé d'obtention d'une substance minérale à partir d'une base comportant une matière minérale, le procédé comprenant l'obtention de la base comportant une quantité prédéterminée de la matière minérale synthétisée par une structure vivante ou une partie de celle-ci,
**caractérisé en ce que** l'obtention de la base comprend la fourniture de la structure vivante et la fourniture d'au moins un microorganisme lactique susceptible de symbiose avec la structure vivante pour la synthèse de la matière minérale de la substance minérale.

2. Procédé selon la revendication 1, dans lequel l'obtention de la base comprend en outre la fourniture d'une substance nutritive, préférablement de carbonate de glycérol et/ou de dérivés de carbonate de glycérol.

3. Procédé selon la revendication 1 ou la revendication 2, dans lequel la structure vivante et le microorganisme lactique sont fournis ensemble de manière à former un complexe biologique.

4. Procédé selon la revendication 1 ou la revendication 2, dans lequel le microorganisme lactique est d'abord fourni puis la structure vivante.

5. Procédé selon l'une des revendications 1 à 4, dans lequel, l'obtention de la base comprend en outre la fourniture d'un substrat de base adapté au développement de la structure vivante et/ou du microorganisme lactique et la mise au contact du substrat de base avec la structure vivante et le microorganisme lactique.

6. Procédé selon la revendication 5, comprenant en outre le nettoyage des surfaces du substrat de base en contact avec le microorganisme lactique par celui-ci ; la synthèse de la matière minérale par la structure vivante, le nettoyage et la synthèse pouvant avoir lieu simultanément ou successivement dans cet ordre.

7. Procédé selon l'une des revendications 1 à 6, comprenant en outre l'inactivation de la base pour obtenir une substance minérale inactivée.

8. Composition minéralisatrice comprenant :
- une structure vivante ou au moins une partie de structure vivante ;
- au moins un microorganisme lactique ; et
- une substance nutritive.

9. Composition minéralisatrice selon la revendication 8 formulée pour une utilisation extemporanée.

10. Substance minérale comprenant :
- une structure vivante ou au moins une partie de celle-ci ;
- au moins un microorganisme lactique ; et
- une matière minérale synthétisée par la structure vivante ou la partie de celle-ci.

11. Substance minérale selon la revendication 10, comprenant en outre un substrat de base adapté à la prolifération de la structure vivante et/ou du microorganisme lactique.

12. Substance minérale selon la revendication 10 ou la revendication 11, dans laquelle la matière minérale, et éventuellement le substrat de base, est présente sous une forme poreuse avec des pores débouchant à l'extérieur de la substance minérale dans lesquels la structure vivante et le microorganisme lactique sont présents.

13. Substance minérale selon la revendication 12, dans laquelle la structure vivante et/ou le microorganisme lactique est/sont inactivé(s).

14. Utilisation d'une combinaison d'une structure vivante et d'un microorganisme lactique en symbiose l'un avec l'autre, comme agent de minéralisation dans un matériau auto-régénérant, le microorganisme lactique étant apte à sécréter de l'acide lactique.

15. Utilisation de la composition minéralisatrice selon l'une des revendications 8 ou 9, ou de la substance minérale selon l'une des revendications 10 à 13 comme agent réparateur dans un matériau auto-régénérant.

## Patentansprüche

1. Verfahren zum Erhalten einer mineralischen Substanz aus einem ein mineralisches Material umfassenden Grundstoff, wobei das Verfahren das Erhalten des Grundstoffs, der eine vorgegebene Menge des mineralischen Materials umfasst, das von einer lebenden Struktur oder einem Teil davon synthetisiert ist,
**dadurch gekennzeichnet, dass** das Erhalten des Grundstoffs, das Bereitstellen der lebenden Struktur und das Bereitstellen mindestens eines Milchsäuremikroorganismus, der für eine Symbiose mit der lebenden Struktur geeignet ist, für die Synthese des mineralischen Materials der mineralischen Substanz umfasst.

2. Verfahren nach Anspruch 1, wobei das Erhalten des Grundstoffs zudem das Bereitstellen eines Nährstoffs, bevorzugt Glycerolcarbonat und/oder Glycerolcarbonatderivate, umfasst.

3. Verfahren nach Anspruch 1 oder Anspruch 2, wobei die lebende Struktur und der Milchsäuremikroorganismus zusammen bereitgestellt werden, so dass sie einen biologischen Komplex bilden.

4. Verfahren nach Anspruch 1 oder Anspruch 2, wobei der Milchsäuremikroorganismus zuerst bereitgestellt wird und anschließend die lebende Struktur.

5. Verfahren nach einem der Ansprüche 1 bis 4, wobei das Erhalten des Grundstoffs zudem die Bereitstellung eines Grundsubstrats, das für die Entwicklung der lebenden Struktur und/oder des Milchsäuremikroorganismus angepasst ist, und das Inkontaktbringen des Grundsubstrats mit der lebenden Struktur und dem Milchsäuremikroorganismus umfasst.

6. Verfahren nach Anspruch 5, das weiter das Reinigen der Oberflächen des Grundsubstrats durch den Milchsäuremikroorganismus, wobei die Oberflächen in Kontakt mit dem Milchsäuremikroorganismus sind; die Synthese des mineralischen Materials durch die lebende Struktur umfasst, wobei das Reinigen und die Synthese gleichzeitig oder aufeinanderfolgend, in dieser Reihenfolge, stattfinden können.

7. Verfahren nach einem der Ansprüche 1 bis 6, das weiter die Inaktivierung des Grundstoffs umfasst, um eine inaktivierte mineralische Substanz zu erhalten.

8. Mineralisierende Zusammensetzung, umfassend:
- eine lebende Struktur oder zumindest einen Teil einer lebenden Struktur;
- mindestens einen Milchsäuremikroorganismus; und
- einen Nährstoff.

9. Mineralisierende Zusammensetzung nach Anspruch 8, die für eine extemporierte Verwendung formuliert ist.

10. Mineralisierende Substanz, umfassend:
- eine lebende Struktur oder zumindest einen Teil davon;
- mindestens einen Milchsäuremikroorganismus; und
- ein mineralisches Material, das von der lebenden Struktur oder dem Teil davon synthetisiert ist.

11. Mineralische Substanz nach Anspruch 10, die weiter ein Grundsubstrat umfasst, das an die Proliferation der lebenden Struktur und/oder des Milchsäuremikroorganismus angepasst ist.

12. Mineralische Substanz nach Anspruch 10 oder Anspruch 11, wobei das mineralische Material, und gegebenenfalls das Grundsubstrat, in poriger Form vorliegen, mit in das Äußere der mineralischen Substanz mündenden Poren, in denen die lebende Struktur und der Milchsäuremikroorganismus vorliegen.

13. Mineralische Substanz nach Anspruch 12, wobei die lebende Struktur und/oder der Milchsäuremikroorganismus inaktiviert ist/sind.

14. Verwendung einer Kombination aus einer lebenden Struktur und einem Milchsäuremikroorganismus, die miteinander in Symbiose stehen, als Mineralisierungsagens in einem autoregenerativen Material, wobei der Milchsäuremikroorganismus fähig ist, Milchsäure zu sekretieren.

15. Verwendung der mineralisierenden Zusammensetzung nach einem der Ansprüche 8 oder 9, oder mineralische Substanz nach einem der Ansprüche 10 bis 13 als reparierendes Agens in einem autoregenerativen Material.

## Claims

1. Method for obtaining a mineral substance from a base comprising mineral matter, the method involving obtaining the base comprising a predetermined quantity of the mineral matter synthesised by a living structure or a portion of the latter,
**characterised in that** obtaining the base comprises providing the living structure and providing at least one lactic acid microorganism suitable for symbiosis with the living structure for the synthesis of the mineral matter of the mineral substance.

2. Method according to claim 1, wherein obtaining the base further comprises providing a nutritive substance, preferably glycerol carbonate and/or derivatives of glycerol carbonate.

3. Method according to claim 1 or claim 2, wherein the living structure and the lactic acid microorganism are provided together in order to form a biological complex.

4. Method according to claim 1 or claim 2, wherein the lactic acid microorganism is provided first, and then the living structure.

5. Method according to one of claims 1 to 4, wherein obtaining the base further comprises providing a base substrate suitable for the development of the living structure and/or of the lactic acid microorganism and bringing the base substrate in contact with the living structure and the lactic acid microorganism.

6. Method according to claim 5, further comprising cleaning the surfaces of the base substrate in contact with the lactic acid microorganism by this microorganism; synthesising the mineral matter by the living structure, cleaning and synthesising taking place simultaneously or successively in this order.

7. Method according to one of claims 1 to 6, further comprising deactivating the base to obtain a deactivated mineral substance.

8. Mineralising composition comprising:
- a living structure or at least a portion of a living structure;
- at least one lactic acid microorganism; and
- a nutritive substance.

9. Mineralising composition according to claim 8 formulated for extemporaneous use.

10. Mineral substance comprising:
- a living structure or at least a portion thereof;
- at least one lactic acid microorganism; and
- mineral matter synthesised by the living structure or the portion thereof.

11. Mineral substance according to claim 10, further comprising a base substrate suitable for the proliferation of the living structure and/or of the lactic acid microorganism.

12. Mineral substance according to claim 10 or claim 11, wherein the mineral matter, and optionally the base substrate, is present in a porous form, with pores, opening outside of the mineral substance, in which the living structure and the lactic acid microorganism are present.

13. Mineral substance according to claim 12, wherein the living structure and/or the lactic acid microorganism is/are deactivated.

14. Use of a combination of a living structure and a lactic acid microorganism in symbiosis with each other as a mineralising agent in a self-regenerating material, the lactic acid microorganism being suitable for secreting lactic acid.

15. Use of the mineralising composition according to one of claims 8 and 9 or of the mineral substance according to one of claims 10 to 13 as a repairing agent in a self-regenerating material.
